## Europäisches Patentamt

## European Patent Office

## Office européen des brevets

(19)

(11) Veröffentlichungsnummer: **0 132 785**
**B1**

(12)

# EUROPÄISCHE PATENTSCHRIFT

(45) Veröffentlichungstag der Patentschrift:
**28.10.87**

(21) Anmeldenummer: **84108497.3**

(22) Anmeldetag: **18.07.84**

(51) Int. Cl.⁴: **A 61 B 5/05,** G 01 N 24/04,
A 61 B 5/04, H 03 H 7/01

(54) Einrichtung zur Gewinnung eines EKG-Signals bei einem Kernspintomographen.

(30) Priorität: **01.08.83 DE 3327731**

(43) Veröffentlichungstag der Anmeldung:
**13.02.85 Patentblatt 85/7**

(45) Bekanntmachung des Hinweises auf die Patenterteilung:
**28.10.87 Patentblatt 87/44**

(84) Benannte Vertragsstaaten:
**DE FR GB NL**

(56) Entgegenhaltungen:
**EP - A - 0 105 550**
**DE - A - 2 717 320**
**FR - A - 2 195 118**
**FR - A - 2 515 026**
**US - A - 3 879 691**
**US - A - 4 095 588**
**US - A - 4 109 223**
**US - A - 4 328 814**

**COMPUTERTOMOGRAPHIE, Band 1, 1981, Seiten 2-10, Georg Thieme Verlag, Stuttgart, DE; A. GANSSEN u.a.: "Kernspin-Tomographie"**
**RÖNTGENSTRAHLEN, Band 49, 1983, Seiten 34-41, DE; J. HEINZERLING: "Technische Fortschritte in der NMR-Tomographie"**
**ELECTROMEDICA, Band 51, Nr. 2, 1983, Seiten 65-72,**

(56) Entgegenhaltungen: (Fortsetzung)
**Erlangen, DE; H. MORNEBURG: "Gesichtspunkte bei der Standortsuche und Planung für ein Magnetom" SIEMENS-BAUTEILE-INFORMATIONEN, Band 5, Heft 4, Oktober 1967, Seiten 115-119, München, DE; H.-P. KAISERSWERTH: "Funk-Entstörung. Teil 3: Funk-Entstörfilter und -geräte"**

(73) Patentinhaber: **Siemens Aktiengesellschaft Berlin und München, Wittelsbacherplatz 2, D-8000 München 2 (DE)**

(72) Erfinder: **Wirth, Axel, Amselstrasse 31, D-8551 Hemhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Einrichtung zur Gewinnung eines EKG-Signals von einem Patienten, der sich in einem Kernspintomographen befindet, welcher mit einer vor Hochfrequenzeinstrahlung schützenden HF-Abschirmung ausgerüstet ist, mit mehreren Elektroden, die zum Anlegen am Patienten und zur Abnahme eines EKG-Ableitsignals vorgesehen sind, mit einem EKG-Verarbeitungsgerät, das aus dem EKG-Ableitsignal das erwähnte EKG-Signal bildet, und mit einem Verbindungskabel, das zwischen den Elektroden und dem EKG-Verarbeitungsgerät angeordnet ist und eine Kabelabschirmung umfasst.

Eine solche Einrichtung zur Gewinnung eines EKG-Signals im Zusammenhang mit der NMR- oder Kernspinresonanz-Tomographie ist aus der Zeitschrift «Röntgenstrahlen», 49, 1983, Seiten 34 bis 41 bekannt. Hierbei wird ein Kernspintomograph verwendet, der mit einer aus dem Magneten herausführbaren HF-Abschirmung arbeitet. Diese HF-Abschirmung schützt vor unerwünschter Beeinflussung der Messung z.B. durch Kurzwellensender. In dieser Literaturstelle ist angegeben, dass mit dem bekannten Kernspintomographen dynamische Untersuchungen durchgeführt werden können. Es lassen sich dabei periodische Vorgänge, wie z.B. die Herzbewegung, im Bild darstellen. Dazu überlagert man mit Hilfe eines gleichzeitig registrierten Elektrokardiogrammes Bilder, die zu gleichen Bewegungsphasen gehören, und erreicht dabei eine akzeptable Bildqualität. In dieser EKG-getriggerten Aufnahmetechnik lassen sich beispielsweise Querschnittsbilder des menschlichen Herzens erzeugen. In dieser Literaturstelle ist nicht im einzelnen beschrieben, wie die getriggerten Herzaufnahmen durchgeführt werden. Insbesondere muss hervorgehoben werden, dass bei der bekannten Einrichtung die HF-Abschirmung als zylindrische Röhre ausgebildet ist, die den Patienten eng umgibt. Es handelt sich also nicht um einen Kernspintomographen, der innerhalb einer Kabine untergebracht ist, die zwecks HF-Abschirmungseffekt mit einer metallischen Schicht oder einem metallischen Geflecht belegt ist.

Aus der Zeitschrift «Computer-Tomographie» 1 (1981), Seiten 2 bis 10, ist ein Kernspin-Tomograph bekannt, bei dem der den Patienten aufnehmende Magnet von einem Faraday'schen Käfig als HF-Abschirmung umgeben ist. Alle Stromzuleitungen für Magnet- und Gradientenspulen sind sorgfältig gefiltert und abgeschirmt. In dieser Literaturstelle wird zwar angegeben, dass bei Untersuchungen des Körperstamms bei den meisten Organen die Bildqualität vermutlich durch die Atembewegung und den Herzschlag beeinträchtigt wird. Es wird jedoch kein Vorschlag in Richtung auf eine EKG-getriggerte Aufnahmetechnik gemacht.

In der Zeitschrift «Spektrum der Wissenschaft» Juli 1982, Seiten 40 bis 55, werden ebenfalls kernspintomographische Prinzipien erläutert und Kernspintomogramme von verschiedenen Partien des menschlichen Körpers gezeigt. In Bild 9 ist ein Kernspintomograph dargestellt, wobei jedoch eine HF-Abschirmung und eine Einrichtung zur Gewinnung eines EKG-Signals weggelassen sind. Andererseits ist bei der Beschreibung zu Bild 12 dargelegt, dass man bei Herzaufnahmen spezielle Bildverfahren benötigt, um die Bewegung des Herzens und die «Belichtungszeit», d.h. den Einsatzpunkt der Aufnahme, zu synchronisieren. Nähere Angaben, wie dieses in der Praxis zu geschehen hat, fehlen.

Es besteht nun das Bedürfnis, auch einen Kernspintomographen, der mit einer vor Hochfrequenzeinstrahlung schützenden HF-Abschirmung in Form eines Faraday-Käfigs ausgerüstet ist, mit einer Einrichtung zur Gewinnung eines EKG-Signals auszustatten. Dies wird ganz allgemein im Hinblick auf die Ableitung von EKG-Signalen von einem Patienten, der gerade einer NMR-Untersuchung unterzogen wird und dabei bezüglich seiner Herztätigkeit überwacht werden soll, als auch speziell im Hinblick auf die EKG-Triggerung des Kernspintomographen von Interesse sein. In Untersuchungen an Patienten in Tomographen mit Faraday-Käfig hat sich nun aber gezeigt, dass die Ableitung des EKG-Triggersignals mit Störungen verbunden ist und dass die Qualität des NMR-Bildes wegen der Massnahmen, die zur Ableitung dieses EKG-Signals getroffen werden, leidet. Insbesondere kann durch eingekoppeltes Rauschen oder aber allein durch das Einbringen von Elektrodenmaterial in den Kernspintomographen die Bildqualität erheblich reduziert werden.

Der Erfindung liegt demzufolge die Aufgabe zugrunde, für einen Kernspintomographen, der mit einer vor Hochfrequenzeinstrahlung schützenden HF-Abschirmung, insbesondere in Form einer Kabine ausserhalb des Magneten, ausgerüstet ist, eine Einrichtung zur Gewinnung eines EKG-Signals anzugeben, wobei eine störungsfreie Ableitung des EKG-Signals sichergestellt sein soll, ohne dass die Bildqualität des NMR-Bildes leidet.

Diese Aufgabe wird bei einer Einrichtung der eingangs genannten Art erfindungsgemäss dadurch gelöst, dass die Kabelabschirmung auf das elektrische Potential der HF-Abschirmung gelegt und nicht am EKG-Verarbeitungsgerät selbst geerdet ist.

Die Erfindung beruht auf der Erkenntnis, dass das von aussen in die NMR-Kabine eingeführte Verbindungskabel als Antenne wirkt. Es bringt über die EKG-Elektroden HF-Signale ins Messfeld, die das NMR-Bild verrauschen. Überraschenderweise lässt sich nun, wie Experimente gezeigt haben, eine sprunghafte Qualitätsverbesserung dadurch erzielen und ein Grossteil der Störungen dadurch beseitigen, dass man den Kabelschirm der EKG-Verlängerungsleitung oder des EKG-Anschlusskabels nicht, wie man wohl normalerweise vorgehen würde, am EKG-Verarbeitungsgerät erdet, sondern mit der Masse der NMR-Kabine verbindet.

2

Eine weitere Bildverbesserung lässt sich dadurch erzielen, dass in das Verbindungskabel ein Filter zum Unterdrücken hochfrequenter Signale eingeschaltet wird. Dieses Filter unterdrückt das Rauschen, das sonst über das Verbindungskabel weitergeleitet würde. Prinzipiell lässt sich dabei ein relativ einfach aufgebautes Filter verwenden. In verbesserter Ausführung sollte jedoch ein Durchführungsfilter verwendet werden, insbesondere ein solches, bei dem in jeder Leitung des Verbindungskabels eine T-Schaltung aus zwei Drosselspulen und einem Kondensator eingeschaltet ist.

Es soll noch besonders betont werden, dass sich mit der vorliegenden Erfindung besonders störungsarm EKG-Triggersignale für den mit Faraday-Käfig ausgerüsteten Kernspintomographen erzeugen lassen, so dass nunmehr auch Herzuntersuchungen mit Kernspintomographen mit Faraday-Käfig bei hervorragender Bildqualität möglich sind.

Allgemein gesprochen lässt sich die Erfindung bei der Gewinnung eines beliebig verarbeiteten EKG-Signals verwenden, nicht nur bei der Bildung von EKG-Triggersignalen.

Weitere Vorteile und Ausgestaltungen der Erfindung ergeben sich aus den Unteransprüchen.

Die Erfindung wird im folgenden anhand von Ausführungsbeispielen näher erläutert. Es zeigen:

Figur 1 eine schematische Darstellung eines Kernspintomographen, der mit einem Faraday-Käfig ausgerüstet ist, und eine EKG-Triggereinrichtung;

Figur 2 ein Durchführungsfilter, das bei der Triggereinrichtung nach Figur 1 eingesetzt werden kann, und

Figur 3 einen an sich bekannten Durchführungskondensator, der bei einem Filter nach Figur 2 verwendet werden kann.

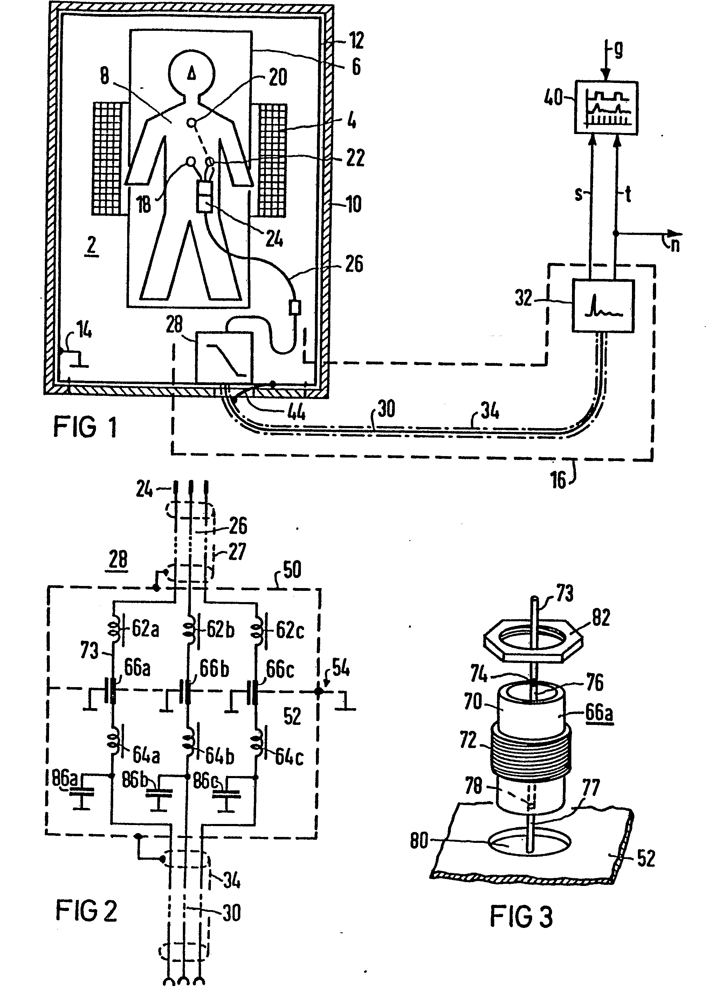

In Figur 1 ist ein Kernspintomograph 2 schematisch veranschaulicht. Dieser Kernspintomograph 2 umfasst ein Magnetsystem 4, in das in bekannter Weise eine Patientenlagerungsplatte 6 einschiebbar ist. Die Patientenlagerungsplatte 6 trägt einen Patienten 8. Der Kernspintomograph 2 ist von einem Gehäuse oder einer NMR-Kabine 10 umgeben. Die NMR-Kabine 10 ist innen mit einer Auskleidung 12 aus Metall versehen. Diese Auskleidung 12 umgibt den Kernspintomographen 2 vollkommen. Sie ist an einer Stelle 14 elektrisch auf Massepotential gelegt und wirkt als Faraday'scher Käfig.

Dem Kernspintomographen 2 ist eine EKG-Messeinrichtung 16 für die Patientenüberwachung zugeordnet. Im vorliegenden Fall handelt es sich insbesondere um eine EKG-Triggereinrichtung 16. Diese Triggereinrichtung 16 umfasst insgesamt drei Elektroden 18, 20 und 22, die in bekannter Weise am Oberkörper des Patienten 8 angelegt sind. Die Anschlusskabel dieser drei Elektroden 18, 20 und 22 führen zu einer Steckverbindung 24, die die abgenommenen EKG-Signale über ein Verbindungskabel 26 weiterleitet. Der eine Stecker der Steckverbindung 24 kann dabei für jede Verbindungsleitung einen eigenen Sicherheitswiderstand in Serie geschaltet enthalten. Als geeigneter Wert für diesen Sicherheits-Widerstand hat sich 10 kOhm erwiesen.

Das Verbindungskabel 26 ist mit einem Filter 28 verbunden, das innerhalb der NMR-Kabine 10 untergebracht ist. Wie später näher erläutert wird, handelt es sich bei diesem Filter 28 bevorzugt um ein Durchführungsfilter.

Das Ausgangssignal des Filters 28 wird über eine Öffnung in der NMR-Kabine 10 und in der Auskleidung 12 mittels eines weiteren Verbindungskabels 30 zu einem EKG-Verarbeitungsgerät 32 weitergeleitet, das ausserhalb der NMR-Kabine 10 angeordnet ist. Die Kabelabschirmung dieses weiteren Verbindungskabels 30 ist besonders eingezeichnet und mit dem Bezugszeichen 34 versehen.

Das EKG-Verarbeitungsgerät 32 ist ein konventionelles EKG-Gerät, das ein den EKG-Verlauf repräsentierendes Signal s sowie ein von der R-Zacke des Herzens abgeleitetes Triggersignal t abgibt. Beide Ausgangssignale s, t werden einem EKG-Schreiber 40 zugeführt, der zusätzlich von einem Gradienten-Triggersignal g beaufschlagt ist. Mit Hilfe des EKG-Schreibers 40 lässt sich das Elektrokardiogramm des Patienten 8 während der Untersuchung im Kernspintomographen 2 ständig überwachen.

Das Triggersignal t, das vom EKG-Verarbeitungsgerät 32 geliefert wird, wird gleichzeitig auf die Steuereinrichtung (nicht gezeigt) des Kernspintomographen 2 gegeben. Es dient dazu, diesen im richtigen Zeitpunkt zu schalten, so dass weitgehend störungsfreie, von der Herzbewegung unbeeinflusste NMR-Bilder zustande kommen. Mit anderen Worten: Bei der EKG-getriggerten Steuerung der NMR-Aufnahme wird das magnetische Gradientenfeld durch die R-Zacke des EKG ausgelöst, d.h. getriggert.

Um Aufnahmen in unterschiedlichen Bewegungsphasen des Herzens machen zu können, z.B. in der Systole oder Dyastole, kann zusätzlich eine (nicht gezeigte) Schaltung vorgesehen sein, mit der das Triggersignal t in bezug zur R-Zacke zeitlich verschoben wird. Eine solche Schaltung ist also ein Zeiteinstellglied, welches zur Optimierung der NMR-Aufnahmen dient.

Von Bedeutung ist es nun, dass im vorliegenden Fall die Kabelabschirmung 34 des weiteren Verbindungskabels 30 auf das elektrische Potential der HF-Abschirmung 12 gelegt ist, dass also die Kabelabschirmung 34 mit dem Faraday'schen Käfig 12 elektrisch leitend verbunden ist. Diese Tatsache ist in Figur 1 durch eine Leitungsverbindung 44 angedeutet. Es soll also betont werden, dass die Abschirmung 34 nicht am EKG-Verarbeitungsgerät 32 selbst geerdet ist, sondern in der Tat mit der Masse der NMR-Kabine 10 verbunden ist. Dadurch lässt sich eine ausgezeichnete Qualität des NMR-Bildes erzielen. Untersuchungen haben nämlich ergeben, dass das NMR-Bild weitgehend verrauscht ist, wenn das Zuleitungskabel 30 mit seiner Abschirmung 34 auf das elektrische Potential des EKG-Verarbeitungsgeräts 32 gelegt ist. Die Antennenwirkung des von aussen in die NMR-Kabine 10 hineingeführten Verbindungskabels 30 lässt sich also durch die Verbindung 44 weitgehend vermeiden.

Eine weitere Verbesserung des Rauschens im NMR-Bild wird dadurch erzielt, dass das Filter 28 vorgesehen ist.

Nach Figur 2 ist dieses Filter 28 speziell als ein Durchführungsfilter ausgebildet. Es reduziert erheblich das Rauschen, das andernfalls über das Zuleitungskabel 30 aufgenommen, in den Faraday'schen Käfig 12 hineingetragen und als Störung auf dem Bildschirm des NMR-Sichtgerätes sichtbar wird.

Das in Figur 2 gezeigte Durchführungsfilter 28 ist im Prinzip ein dreifaches Filter, das eine hohe Dämpfung im Megahertz-Bereich und eine niedere Dämpfung im Niederfrequenzbereich aufweist. Es dient also dazu, hochfrequente Signale zu unterdrücken.

Nach Figur 2 ist das Durchführungsfilter 28 in einem Gehäuse 50 aus Metall untergebracht, das mittig mit einer metallischen Trennwand 52 versehen ist. Das Gehäuse 50 und die Trennwand 52 sind an der Stelle 54 auf das elektrische Potential des Faraday'schen Käfigs 12 und damit auf NMR-Masse gelegt. Das Durchführungsfilter 28 ist zwischen den beiden Verbindungskabeln 26 und 30 angeordnet. Wie aus Figur 2 ersichtlich, ist die Abschirmung 27, 34 jedes dieser beiden Verbindungskabel 26, 30 ebenfalls auf NMR-Masse gelegt. Jede der drei Leitungen des Kabels 26, 30 enthält dabei eine T-Schaltung aus zwei Drosselspulen 62a, 64a und 62b, 64b sowie 62c, 64c sowie einem Durchführungskondensator 66a, 66b, 66c. Die Durchführungskondensatoren 66a, 66b, 66c sind jeweils durch die Trennwand 52 hindurchgeführt und dabei mit einem Belag auf deren elektrisches Potential gelegt.

In Figur 3 ist angedeutet, in welcher Weise dies beispielsweise beim Durchführungskondensator 66a geschehen ist. Der Durchführungskondensator 66a besteht im Prinzip aus einem Keramikröhrchen 70, das im mittleren Bereich mit einem metallischen Belag mit Gewinde 72 versehen ist. Die Zuführungsleitung 73 wird an einer Lötstelle 74 mit einem innerhalb des Röhrchens 70 befindlichen Draht 76 verlötet, und entsprechend wird die weiterführende Leitung 77 an einer Lötstelle 78 am anderen Ende mit dem Drähtchen 76 verlötet. Nach der Montage befindet sich das Gewinde 72 in einer Öffnung 80 der Trennwand 52, und der Kondensator 66a kann mittels zweier Muttern, von denen nur die obere Mutter 82 gezeigt ist, in dieser Position in der Trennwand 52 fixiert werden. Die Kapazität des Durchführungskondensators 66a wird dabei durch das Drähtchen 76 einerseits und den umgebenden metallischen Belag 72 andererseits gebildet. Durch die Befestigung mittels der Muttern ist gleichzeitig ein elektrischer Anschluss an das Potential der Trennwand 52 und damit an NMR-Masse gewährleistet.

In einer realisierten Ausführungsform konnte mit einem Durchführungsfilter 28 gemäss Figur 2 eine Dämpfung von etwa 85 dB im MHz-Bereich erzielt werden. Bei dieser Ausführungsform besassen die einzelnen Drosseln 62a, 62b, 62c sowie 64a, 64b, 64c jeweils eine Induktivität von 6 800 μH und die Durchführungskondensatoren 66a, 66b, 66c jeweils eine Kapazität von 10 nF.

Aus Figur 2 ist ersichtlich, dass das Durchführungsfilter 28 ausgangsseitig jeweils an jeder Leitung noch einen Kondensator 86a, 86b, 86c aufweist. Diese Kondensatoren 86a, 86b, 86c sind jeweils mit einem Belag an der Ausgangsleitung und mit dem anderen Belag an Masse angeschlossen. Sie dienen zur Verbesserung der Dämpfung des Durchführungsfilters 28 im Mittelfrequenzbereich.

Um gute Triggermöglichkeiten zu schaffen, vor allem bei einem gestörten EKG, ist es wichtig, eine deutliche R-Zacke zu haben, die im EKG-Verarbeitungsgerät 32 einwandfrei verarbeitet werden kann. Die Untersuchungen haben gezeigt, dass hierfür am günstigsten die Brustwandableitung ist, wie sie in Figur 1 gezeigt ist. Hierbei wird vorteilhafterweise die Zuleitung zur oberen Elektrode 22 über die rechts angelegte Elektrode 22 geführt und dort fixiert. Dadurch wird die Fläche, die als Störungen auffangende Induktionsschleife in Betracht kommt, klein gehalten, und es wird gute Signaltreue bezüglich der EKG-Kurvenform erzielt. Die Elektroden 20, 22 sind hierbei die aktiven Elektroden oder Messelektroden, und die Elektrode 18 ist die neutrale Elektrode. Die Zuleitung der einen aktiven Elektrode 20 ist also über die andere aktive Elektrode 22 gelegt.

Es soll noch betont werden, dass die üblichen Telefonfilter für eine Anpassung von 600 Ohm ausgelegt sind. Wegen der Hochohmigkeit des EKG lassen sich solche Telefonfilter nicht als Filter 28 verwenden. Bei Telefonfiltern ist die Dämpfung im hier interessierenden Frequenzbereich zu gross, so dass das EKG-Triggersignal t nicht mehr ausgelöst werden kann.

Das Kabel 26, 30 enthält drei parallel geführte Leitungen und ist aufgrund seiner Kapazität für die Hochfrequenz niederohmig. Dasselbe gilt für die Hauptimpedanz des Patienten. Aus Sicherheitsgründen wurde daher im Stecker des Patientenkabels in der Steckverbindung 24 der erwähnte Widerstand von 10 kOhm in jede der drei Leitungen eingesetzt. Dies ist eine reine Schutzmassnahme.

## Patentansprüche

1. Einrichtung zur Gewinnung eines EKG-Signals von einem Patienten (8), der sich in einem Kernspintomographen (2) befindet, welcher mit einer vor Hochfrequenzeinstrahlung schützenden HF-Abschirmung (12) ausgerüstet ist, mit mehreren Elektroden (18, 20, 22), die zum Anlegen am Patienten (8) und zur Abnahme eines EKG-Ableitsignals vorgesehen sind, mit einem EKG-Verarbeitungsgerät (32), das aus dem EKG-Ableitsignal das erwähnte EKG-Signal (s, t) bildet, und mit einem Verbindungskabel (26, 30), das zwischen den Elektroden (18, 20, 22) und dem EKG-Verarbeitungsgerät (32) angeordnet ist und eine Kabelabschirmung (27, 34) umfasst, dadurch gekennzeichnet, dass die Kabelabschirmung (27, 34) auf das elektrische Potential der HF-Abschirmung (12) gelegt und nicht am EKG-Verarbeitungsgerät (32) selbst geerdet ist.

2. Einrichtung nach Anspruch 1, dadurch gekennzeichnet, dass in das Verbindungskabel (26, 30) ein Filter (28) zum Unterdrücken hochfrequenter Signale eingeschaltet ist.

3. Einrichtung nach Anspruch 2, dadurch gekennzeichnet, dass das Filter (28) ein Durchführungsfilter ist.

4. Einrichtung nach Anspruch 2 oder 3, dadurch gekennzeichnet, dass in jede Leitung des Verbindungskabels (26, 30) als Filter (28) eine T-Schaltung aus zwei Drosselspulen (62, 64) und einem Kondensator (66) geschaltet ist.

5. Einrichtung nach Anspruch 4, dadurch gekennzeichnet, dass an den Ausgang jeder T-Schaltung ein Kondensator (86) mit seinem einen Belag angeschlossenen ist, wobei der andere Belag auf das elektrische Potential der HF-Abschirmung (12) des Kernspintomographen (2) gelegt ist.

6. Einrichtung nach einem der Ansprüche 3, 4 oder 5, dadurch gekennzeichnet, dass das Durchführungsfilter (28) in einem Gehäuse (50) untergebracht ist, das auf dem elektrischen Potential der HF-Abschirmung (12) des Kernspintomographen (2) liegt, und dass dieses Gehäuse (50) eine metallische Trennwand (52) enthält, durch die Durchführungskondensatoren (66a, 66b, 66c) gesteckt sind, die mit der Trennwand (52) elektrisch leitend verbunden sind.

7. Einrichtung nach einem der Ansprüche 3 bis 6, dadurch gekennzeichnet, dass das EKG-Verarbeitungsgerät (32) ausserhalb der HF-Abschirmung (12) des Kernspintomographen (2) angeordnet ist.

8. Einrichtung nach einem der Ansprüche 1 bis 7, dadurch gekennzeichnet, dass eine erste Leitung des Verbindungskabels (26), die mit einer ersten (20) der Elektroden (18, 20, 22) verbunden ist, über eine zweite (22) der Elektroden geführt (18, 20, 22) ist und auf dieser zweiten Elektrode (22) unter elektrischer Trennung mechanisch fixiert ist.

9. Einrichtung nach einem der Ansprüche 1 bis 8, dadurch gekennzeichnet, dass das EKG-Verarbeitungsgerät (32) in an sich bekannter Weise aus dem EKG-Ableitsignal ein EKG-Triggersignal (r) bildet, das zum Triggern des Kernspintomographen vorgesehen ist.

**Revendications**

1. Dispositif pour obtenir un signal électrocardiographique d'un patient (8), qui se situe dans un appareil de tomographie a résonance magnétique nucléaire (2) pourvu d'un blindage HF (12) assurant une protection vis-à-vis de la pénétration d'un rayonnement à haute fréquence, et comportant plusieurs électrodes (18, 10, 22) qui sont prévues pour être appliquées sur le patient (8) et pour prélever un signal dérivé de l'électrocardiogramme, un appareil (32) de traitement du signal électrocardiographique, qui forme, à partir du signal dérivé de l'électrocardiogramme, le signal électrocardiographique (s, t) mentionné, et un câble de liaison (26, 30), qui est disposé entre les électrodes (18, 20, 22) et l'appareil (32) de traitement du signal électrocardiographique, et qui comporte un blindage (27, 34), caractérisé par le fait que le blindage (27, 34) du câble est placé au potentiel électrique du blindage HF (12) et n'est pas raccordé à la terre au niveau de l'appareil (32) de traitement du signal électrocardiographique, lui-même.

2. Dispositif suivant la revendication 1, caractérisé par le fait qu'un filtre (28) servant à supprimer les signaux à haute fréquence est branché dans le câble de liaison (26, 30).

3. Dispositif suivant la revendication 2, caractérisé par le fait que le filtre (28) est un filtre de traversée.

4. Dispositif suivant la revendication 2 ou 3, caractérisé par le fait qu'un circuit en T formé de deux bobines d'arrêt (62, 64) et d'un condensateur (66) est branché, en tant que filtre (28), dans chaque conducteur du câble de liaison (26, 30).

5. Dispositif suivant la revendication 4, caractérisé par le fait qu'une armature d'un condensateur (86) est raccordée à la sortie de chaque circuit en T, l'autre armature étant placée au potentiel électrique du blindage HF (12) du tomographe à résonance magnétique nucléaire (2).

6. Dispositif suivant l'une des revendications 3, 4 ou 5, caractérisé par le fait que le filtre de traversée (28) est logé dans un boîtier (50), qui est placé au potentiel électrique du blindage HF (12) du tomographe à résonance magnétique nucléaire (2), et que le boîtier (50) contient une paroi de séparation métallique (52), à travers laquelle sont enfichés les condensateurs de traversée (66a, 66b, 66c) qui sont reliés de façon électriquement conductrice à la paroi de séparation (52).

7. Dispositif suivant l'une des revendications 3 à 6, caractérisé par le fait que, l'appareil (32) de traitement du signal électrocardiographique est disposé à l'extérieur du blindage HF (12) du tomographe à résonance magnétique nucléaire (2).

8. Dispositif suivant l'une des revendications 1 à 7, caractérisé par le fait qu'un premier conducteur du câble de liaison (26), qui est relié à une première (20) des électrodes (18, 20, 22), s'étend jusqu' à une seconde (22) des électrodes (18, 20, 22) et est fixé mécaniquement à cette seconde électrode (22), moyennant l'établissement d'une séparation électrique.

9. Dispositif suivant l'une des revendications 1 à 8, caractérisé par le fait que l'appareil (32) de traitement du signal électrocardiographique forme de façon connue en soi, à partir du signal dérivé de l'électrocardiogramme, un signal (c) de déclenchement par l'électrocardiogramme, qui est destiné à déclencher le tomographe à résonance magnétique nucléaire.

**Claims**

1. A device for obtaining an ECG-signal from a patient (8) who is arranged in a nuclear magnetic resonance tomograph (2) which is equipped with an HF-screen (2) which provides a protection from high-frequency irradiation, with a plurality of electrodes (18, 20, 22) which serve for connection to the patient (8) and to obtain an ECG-acquired signal, with an ECG-processing device (32) which forms the aforementioned ECG-signal (x, t) from the ECG-acquired signal, and with a connection cable (26, 30) which is arranged between the electrodes (18, 20, 22) and the ECG-processing device (32) and which comprises a cable screen (27, 34) characterised in that the cable screen (27, 34) is connected to the electrical potential of the HF-screen (12) and is not earthed to the ECG-processing device (32) itself.

2. A device as claimed in claim 1, characterised in that a filter (28) is connected into the connection cable (26, 30) in order to suppress high-frequency signals.

3. A device as claimed in claim 2, characterised in that the filter (28) is a feedthrough filter.

4. A device as claimed in claim 2 or 3, characterised in that a T-circuit comprising two choke coils (62, 64) and a capacitor (66) is connected into each line of the connection cable (26, 30) as filter (28).

5. A device as claimed in claim 4, characterised in that the output of each T-circuit is connected to the first coating of a capacitor (86), whose other coating is connected to the electrical potential of the HF-screen (12) of the nuclear magnetic resonance tomograph (2).

6. A device as claimed in one of the claims 3, 4 or 5, characterised in that the feedthrough filter (28) is accommodated in a housing (50) which is connected to the electrical potential of the HF-screen (12) of the nuclear magnetic resonance tomograph (2), and that this housing (50) includes a metallic partition wall (52) through which the feedthrough capacitors (66a, 66b, 66c) are passed, where the feedthrough capacitors are electrically conductively connected to the partition wall (52).

7. A device as claimed in one of the claims 3 to 6, characterised in that the ECG-processing device (32) is arranged outside the HF-screen (12) of the nuclear magnetic resonance tomograph (2).

8. A device as claimed in one of the claims 1 to 7, characterised in that a first conductor of the connection cable (26), which is connected to a first (20) of the electrodes (18, 20, 22) extends via a second (22) of the electrodes (18, 20, 22) and is mechanically fixed to this second electrode (22) whilst being electrically isolated therefrom.

9. A device as claimed in one of the claims 1 to 8, characterised in that in a manner known per se, from the ECG-acquired signal the ECG-processing device (32) forms an ECG-trigger signal (t) which serves to trigger the nuclear magnetic resonance tomograph.

FIG 1

FIG 2

FIG 3